# EUROPEAN PATENT SPECIFICATION

(11) **EP 1 467 728 B1**
(45) Date of publication and mention of the grant of the patent: **22.08.2007**
(21) Application number: 03704413.8
(22) Date of filing: 16.01.2003
(51) Int. Cl.: A61K 31/41, A61K 31/192, A61K 31/216, A61P 9/12, A61P 9/10, A61P 13/12, A61P 25/28, A61P 3/10

(54) **PHARMACEUTICAL COMPOSITIONS COMPRISING VALSARTAN AND NEP INHIBITORS**
PHARMAZEUTISCHE ZUSAMMENSETZUNG ENTHALTEND VALSARTAN UND NEP-INHIBITOREN
COMPOSITIONS PHARMACEUTIQUES A BASE DE VALSARTAN ET D'INHIBITEURS NEP

(30) Priority: 17.01.2002 US 349660 P
(43) Date of publication of application: 20.10.2004
(73) Proprietor: Novartis AG, 4056 Basel (CH); Novartis Pharma GmbH, 1230 Wien (AT)
(72) Inventor: WEBB, Randy, Lee, Flemington, NJ 08822 (US); KSANDER, Gary, Michael, Amherst, New Hampshire 03031 (US)
(74) Representative: Morris, Clive Sydney
(86) International application number: PCT/EP2003/000415
(87) International publication number: WO 2003/059345

(56) References cited:
- EP-A- 0 498 361
- EP-A- 0 726 072
- WO-A-01/74348
- WO-A-02/06253
- WO-A-02/092622
- US-A- 5 217 996

## Description

Angiotensin II interacts with specific receptors on the surface of the target cell. It has been possible to identify receptor subtypes that are termed e.g. AT 1- and AT 2-receptors. In recent times great efforts have been made to identify substances that bind to the AT 1-receptor. Such active ingredients are often termed angiotensin II antagonists. Because of the inhibition of the AT 1-receptor such antagonists can be used e.g. as antihypertensives or for the treatment of congestive heart failure, among other indications. Angiotensin II antagonists are therefore understood to be those active ingredients which bind to the AT 1-receptor subtype.

Inhibitors of the renin angiotensin system are well known drugs that lower blood pressure and exert beneficial actions in hypertension and in congestive heart failure as described, for example, in N. Eng. J. Med. 316, 23 (1987) p. 1429-1435. A large number of peptide and non-peptide inhibitors of the renin angiotensin system are known, the most widely studied being the ACE inhibitors, which includes the drugs captopril, enalapril, lisinopril, benazepril and spirapril. Although a major mode of action of ACE inhibitors involves prevention of formation of the vasoconstrictor peptide Ang II, it has been reported in Hypertension, 16, 4 (1990) p. 363-370 that ACE cleaves a variety of peptide substrates, including the vasoactive peptides bradykinin and substance P. Prevention of the degradation of bradykinin by ACE inhibitors has been demonstrated, and the activity of the ACE inhibitors in some conditions has been reported in Circ. Res., 66, 1 (1990) p. 242-248 to be mediated by elevation of bradykinin levels rather than inhibition of Ang II formation. Consequently, it cannot be presumed that the effect of an ACE inhibitor is due solely to prevention of angiotensin formation and subsequent inhibition of the renin angiotensin system.

Neutral endopeptidase (EC 3.4.24.11; enkephalinase; atriopeptidase; NEP) is a zinc-containing metalloprotease that cleaves a variety of peptide substrates on the amino terminal side of aromatic amino acids. See Biochem. J., 241, (1987) p. 237-247. Substrates for this enzyme include, but are not limited to, atrial natriuretic factors (ANF, also known as ANP), brain natriuretic peptide (BNP), met and leu enkephalin, bradykinin, neurokinin A, and substance P.

ANPs are a family of vasodilator, diuretic and antihypertensive peptides which have been the subject of many recent reports in the literature, for example Annu. Rev. Pharm. Tox., 29, (1989) p. 23-54. One form, ANF 99-126, is a circulating peptide hormone which is released from the heart during conditions of cardiac distension. The function of ANF is to maintain salt and water homeostasis as well as to regulate blood pressure. ANF is rapidly inactivated in the circulation by at least two processes: a receptor-mediated clearance reported in Am. J. Physiol-, 256 (1989) p. R469-R475 and an enzymatic inactivation via NEP reported in Biochem. J., 243 (1987) p. 183-187. It has been previously demonstrated that inhibitors of NEP potentiate the hypotensive, diuretic, natriuretic and plasma ANF responses to pharmacological injection of ANF in experimental animals. The potentiation of ANF by two specific NEP inhibitors is reported by Sybertz et al- in J. Pharmacol. Exp. Ther. 250, 2 (1989) p. 624-631 and in Hypertension, 15, 2 (1990) p. 152-161, while the potentiation of ANF by NEP in general was disclosed in U.S. Patent No. 4,749,688. In U.S. Patent No. 4,740, 499 Olins disclosed the use of thiorphan and kelatorphan to potentiate atrial peptides. Moreover, NEP inhibitors lower blood pressure and exert ANF-like effects such as diuresis and increased cyclic guanosine 3',5'-monophosphate (cGMP) excretion in some forms of experimental hypertension. The antihypertensive action of NEP inhibitors is mediated through ANF because antibodies to ANF will neutralize the reduction in blood pressure.

Prolonged and uncontrolled hypertensive vascular disease ultimately leads to a variety of pathological changes in target organs such as the heart and kidney. Sustained hypertension can lead as well to an increased occurrence of stroke. Therefore, there is a strong need to evaluate the efficacy of antihypertensive therapy, an examination of additional cardiovascular endpoints, beyond those of blood pressure lowering, to get further insight into the benefits of combined treatment.

The nature of hypertensive vascular diseases is multifactorial. Under certain circumstances, drugs with different mechanisms of action have been combined. However, just considering any combination of drugs having different mode of action does not necessarily lead to combinations with advantageous effects. Accordingly, there is a need for more efficacious combination therapy which has less deleterious side effects.

WO 01/74348 discloses pharmaceutical compositions comprising omapatrilat or gemopatrilat and an antihypertensive agent. An example of the antihypertensive agent is Valsartan. These compositions are used to treat systolic hypertension.

EP-A-0498361 and EP-A-0726072 disclose pharmaceutical compositions comprising certain NEP inhibitors and an AT-1 inhibitor. These pharmaceutical compositions are used to treat e.g. hypertension or renal disorders in one aspect, the present invention relates to a pharmaceutical composition comprising
(i) the AT 1-antagonist valsartan or a pharmaceutically acceptable salt thereof and
(ii) N-(3-carboxy-1 -oxopropyl)-(4S)-p-pheny)phenylmethyl)-4-amino-2R-methylbutanoic acid ethyl ester or N-(3-carboxy-1-oxopropyl)-(4S)-p-phenylphenyimethyl)-4-amino-2R-methylbutanoic acid or a pharmaceutically acceptable salt thereof
and a pharmaceutically acceptable carrier.

In another aspect, the present invention relates to a kit comprising in separate containers in a single package pharmaceutical compositions comprising in one container a pharmaceutical composition comprising N-(3-carboxy-1-oxopropyl)-(4S)-p-phenylphenylmethyl)-4-amino-2R-methylbutanoic acid ethyl ester or N-(3-carboxy-1-oxopropyl)-(4S)-p-phenylphenylmethyl)-4-amino-2R-methylbutanoic acid and in a second container a pharmaceutical composition comprising valsartan.

In another embodiment of the invention the present invention relates to a pharmaceutical composition as described above and a diuretic, especially hydrochlorothiazide.

Valsartan is the AT 1 receptor antagonist (S) -N-(1-carboxy-2-methyl-prop-1-yl)-N-pentanoyl-N-[2;(1H-tetrazol-5-yl)biphenyl-4-yl-methyl]amine of formula (1) and is disclosed in EP 0443983 A and United States Patent 5,399,578, the disclosures of 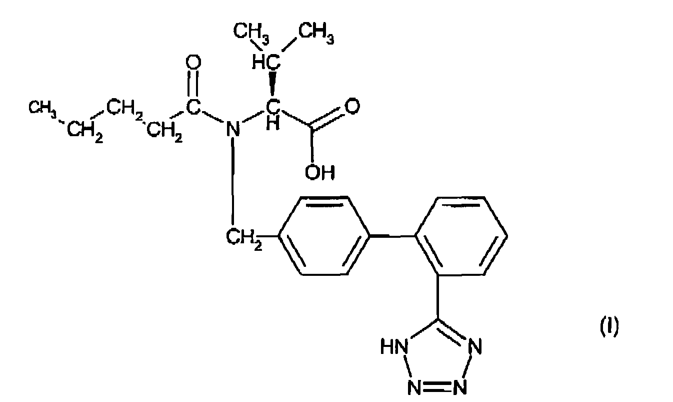 which are incorporated herein in their entirety as if set forth herein.

The NEP inhibitors within the scope of the present invention, namely N-carboxy-1-oxopropyl)-(4S)-p-phenylphenylmethyl)-4-amino-2R-methylbutanoic acid ethyl ester and N-(3-carboxy-1-oxopropyl)-(4S)-p-phenylphenylmethyl)-4-amino-2R-methylbutanoic acid or in each case a pharmaceutically acceptable salt thereof, are disclosed in U.S. Patent No. 5,217,996.

A diuretic is, for example, a thiazide derivative selected from the group consisting of chlorothiazide, hydrochlorothiazide, methylclothiazide, and chlorothalidon. The most preferred is hydrochlorothiazide.

The compounds to be combined can be present as pharmaceutically acceptable salts. If these compounds have, for example, at least one basic center, they can form acid addition salts. Corresponding acid addition salts can also be formed having, if desired, an additionally present basic center. The compounds having at least one acid group (for example COOH) can also form salts with bases. Corresponding internal salts may furthermore be formed, if a compound comprises e.g. both a carboxy and an amino group.

With respect to N-(3-carboxy-1-oxopropyl)-(4S)-p-phenylphenymethyl)-4-amino-2R-methylbutanoic acid ethyl ester, preferred salts include the sodium salt disclosed in U.S. Patent No. 5,217,996, the triethanolamine salt and the tris(hydroxymethyl)aminomethane salt. Preparation of the triethanolamine salt and the tris(hydroxymethyl)aminomethane salt may be carried out as follows:
Triethanolamine - To N-(3-carboxy-1-oxopropyl)-(4S)-p-phenylphenylmethyl)-4-amino-2R-methylbutanoic acid ethyl ester (349 mg, 0.848 mmol) is added 5 ml of ethyl ether and 0-113 ml (0.848 mmol) of triethanolamine in 1 ml of ethyl acetate. The solid was collected and dried melting at 69-71 °C 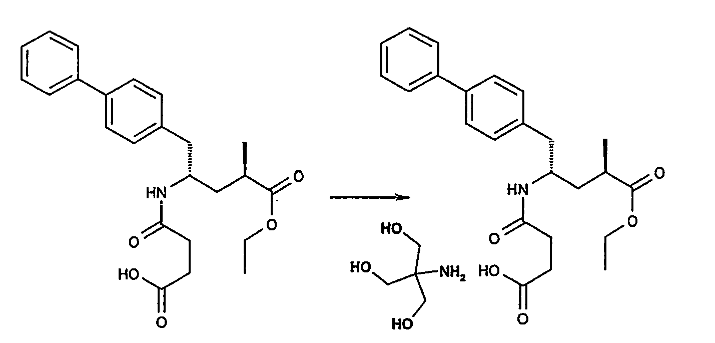
Tris(hydroxymethyl) aminomethane - To N-(3-carboxy-1-oxopropyl)-(4S)-p-phenylphenylmethyl)-4-amino-2R-methylbutanoic acid ethyl ester (3.2 g (7.78 mmol) is added 32 ml of ethyl acetate and 940 mg (7.78 mmol) tris(hydroxymethyl)aminomethane. The suspension is diluted with 45 ml of ethyl acetate and refluxed overnight (-20 hr). The reaction is cooled to 0°C, filtered, solid washed with ethyl acetate and dried melting at 114-115°C.
The salts of N-(3-carboxy-1-oxopropyl)-(4S)-p-phenylphenylmethyl)-4-amino-2R-methylbutanoic acid ethyl ester formed with triethanolamine and tris(hydroxymethyl) aminomethane are novel and can be used as NEP inhibitors. Another embodiment of the present invention are said new salts, their use as NEP inhibitors, especially for preventing and treating of conditions and disease associated with the inhibition on NEP, pharmaceutical composition comprising these salts and their combination with valsartan, especially for the treatment of conditions and diseases as disclosed for the combinations of the present invention hereinbefore or hereinafter.

It has surprisingly been found that, a combination of valsartan and a NEP inhibitor achieves greater therapeutic effect than the administration of valsartan, ACE inhibitors or NEP inhibitors alone and promotes less angioedema than is seen with the administration of a vasopeptidase inhibitor alone. Greater efficacy can also be documented as a prolonged duration of action. The duration of action can be monitored as either the time to return to baseline prior to the next dose or as the area under the curve (AUC) and is expressed as the product of the change in blood pressure in millimeters of mercury (change in mmHg) and the duration of the effect (minutes, hours or days).

Further benefits are that lower doses of the individual drugs to be combined according to the present invention can be used to reduce the dosage, for example, that the dosages need not only often be smaller but are also applied less frequently, or can be used to diminish the incidence of side effects. The combined administration of valsartan or a pharmaceutically acceptable salt thereof and a NEP inhibitor or a pharmaceutically acceptable salt thereof results in a significant response in a greater percentage of treated patients, that is, a greater responder rate results, regardless of the underlying etiology of the condition. This is in accordance with the desires and requirements of the patients to be treated.

It can be shown that combination therapy with valsartan and a NEP inhibitor results in a more effective antihypertensive therapy (whether for malignant, essential, reno-vascular, diabetic, isolated systolic, or other secondary type of hypertension) through improved efficacy as well as a greater responder rate.

The structure of the active agents identified by generic or tradenames or code nos. may be taken from the actual edition of the standard compendium "The Merck Index" or from databases, e.g. Life Cycle Patents International (e.g. IMS World Publications). The corresponding content thereof is hereby incorporated by reference. Any person skilled in the art is fully enabled to identify the active agents and, based on these references, likewise enabled to manufacture and test the pharmaceutical indications and properties in standard test models, both in vitro and in vivo.

The person skilled in the pertinent art is fully enabled to select a relevant test model to prove the efficacy of a combination of the present invention in the hereinbefore and hereinafter indicated therapeutic indications.

Representative studies are carried out with a combination of valsartan and N-(3-carboxy-1-oxopropyl)-(4S)-p-phenylphenylmethyl)-4-amino-2R-methylbutanoic acid ethyl ester, e.g. applying the following methodology:
Drug efficacy is assessed in various animal models including the deoxycorticosterone acetate - salt rat (DOCA-salt) and the spontaneously hypertensive rat (SHR), either maintained on a normal salt diet or with salt loading (4-8% salt in rat chow or 1% NaCl as drinking water).
The DOCA-salt test model utilizes either an acute or chronic study protocol. An acute study procedure involves assessment of the effects of various test substances over a six-hour experimental period using rats with indwelling femoral arterial and venous catheters. The Acute Study Procedure evaluates test substances for their ability to reduce blood pressure during the established phase of DOCA-salt hypertension. In contrast, the Chronic Study Procedure assesses the ability of test substances to prevent or delay the rise in blood pressure during the development phase of DOCA-salt hypertension. Therefore, blood pressure will be monitored in the chronic study procedure by means of a radiotransmitter. The radiotransmitter is surgically implanted into the abdominal aorta of rats, prior to the initiation of DOCA-salt treatment and thus, prior to the induction of hypertension. Blood pressure is chronically monitored for periods of up 6 weeks (approximately one week prior to DOCA-salt administration and for 5 weeks thereafter).
Rats are anesthetized with 2-3% isoflurane in oxygen inhalant followed by Amytal sodium (amobarbital) 100 mg/kg, ip. The level of anesthesia is assessed by a steady rhythmic breathing pattern.

### Acute study procedure:

Rats undergo a unilateral nephrectomy at the time of DOCA implantation. Hair is clipped on the left flank and the back of the neck and scrubbed with sterile alcohol swabs and povidone/iodine. During surgery rats are placed on a heating pad to maintain body temperature at 37 degrees C.

A 20mm incision is made through the skin and underlying muscle to expose the left kidney. The kidney is freed of surrounding tissue, exteriorized and two ligatures (3-0 silk) are tied securely around the renal artery and vein proximal to their juncture with the aorta. The renal artery and vein are then severed and the kidney removed. The muscle and skin wounds are closed with 4-0 silk suture and stainless steel wound clips, respectively. At the same time, a 15mm incision is made on the back of the neck and a 3-week-release pellet (Innovative Research of America, Sarasota, Florida) containing deoxycorticosterone acetate (100 mg/kg) is implanted subcutaneously. The wound is then closed with stainless-steel clips and both wounds are treated with povidone/iodine; the rats are given a post-surgical intramuscular injection of procaine penicillin G (100,000 U) and buprenorphine (0.05 - 0.1 mg/kg) s.c. The rats are immediately placed on 1% NaCl + 0.2% KCI drinking water; this treatment continues for at least 3 weeks at which time the animals have become hypertensive and available for experimentation.

Forty-eight hours prior to experimentation, animals are anesthetized with isoflurane and catheters are implanted in the femoral artery and vein for measuring arterial pressure, collection of blood, and administration of test compounds. Rats are allowed to recover for 48 hours while tethered in a Plexiglas home cage, which also serves as the experimental chamber.

### Chronic study procedure:

This procedure is the same as above except that rats are implanted with a radiotransmitter, 7-10 days prior to the unilateral nephrectomy and initiation of DOCA and salt. In addition, rats do not undergo surgery for placement of femoral arterial and venous catheters. Radiotransmitters are implanted as described in M.K. Bazil, C. Krulan and R.L. Webb. Telemetric monitoring of cardiovascular parameters in conscious spontaneously hypertensive rats. J.Cardiovasc. Pharmacol. 22: 897-905, 1993.

Protocols are then set-up on the computer for measurement of blood pressure, heart rate, etc, at predetermined time points. Baseline data is collected at various time points and over various time intervals. For example, baseline or pre-dose values usually consist of data collection and averaging over 3 consecutive, 24-hour time periods prior to drug administration.

Blood pressure, heart rate and activity are determined at various pre-selected time points before, during, and after drug administration. All measurements are performed in unrestrained and undisturbed animals. The maximum study time, determined by battery life, could be as long as nine months. For studies of this duration, rats are dosed orally (1-3 ml/kg vehicle), no more than twice daily or drug is administered via the drinking water or mixed with food. For studies of a shorter duration, that is, up to 8 weeks, drugs are given via subcutaneously implanted osmotic minipumps. Osmotic minipumps are selected based on drug delivery rate and time. Valsartan dosages range from 1 to 10 mg/kg/day and N-(3-carboxy-1-oxopropyl)-(4S)-p-phenylphenylmethyl)-4-amino-2R-methylbutanoic acid ethyl ester range from 10 to 50 mg/kg/day.

Additionally, SHR are utilized to study the effects of valsartan in combination with N-(3-carboxy-1-oxopropyl)-(4S)-p-phenylphenylmethyl)-4-amino-2R-methylbutanoic acid ethyl ester. The hypertensive background of the SHR is modified either by chronic salt loading in an effort to suppress the renin angiotensin system (RAS) or chronic salt depletion to activate the RAS in the SHR. These manipulations will be carried out to more extensively evaluate the efficacy of the various test substances. Experiments performed in spontaneously hypertensive rats (SHR) are supplied by Taconic Farms, Germantown, New York (Tac:N(SHR)fBR). A radiotelemetric device (Data Sciences International, Inc., St. Paul, Minnesota) is implanted into the lower abdominal aorta of all test animals between the ages of 14 to 16 weeks of age. All SHR are allowed to recover from the surgical implantation procedure for at least 2 weeks prior to the initiation of the experiments. Cardiovascular parameters are continuously monitored via the radiotransmitter and transmitted to a receiver where the digitized signal is then collected and stored using a computerized data acquisition system. Blood pressure (mean arterial, systolic and diastolic pressure) and heart rate are monitored in conscious, freely moving and undisturbed SHR in their home cages. The arterial blood pressure and heart rate are measured every 10 minutes for 10 seconds and recorded. Data reported for each rat represent the mean values averaged over a 24 hour period and are made up of the 144-10 minute samples collected each day. The baseline values for blood pressure and heart rate consist of the average of three consecutive 24 hour readings taken prior to initiating the drug treatments. All rats are individually housed in a temperature and humidity controlled room and are maintained on a 12 hour light dark cycle.

In addition to the cardiovascular parameters, weekly determinations of body weight also are recorded in all rats. Treatments are administered in the drinking water, via daily oral gavage or in osmotic minipumps as stated above. If given in drinking water, water consumption is measured five times per week. Valsartan and N-(3-carboxy-1-oxopropyl)-(4S)-p-phenylphenylmethyl)-4-amino-2R-methylbutanoic acid ethyl ester doses for individual rats are then calculated based on water consumption for each rat, the concentration of drug substance in the drinking water, and individual body weights. All drug solutions in the drinking water are made up fresh every three to four days. Typical dosages for valsartan in drinking water range from 3 to 30 mg/kg/day whereas the dosage of N-(3-carboxy-1-oxopropyl)-(4S)-p-phenylphenylmethyl)-4-amino-2R-methylbutanoic acid ethyl ester is highly dependent upon the specific agent used. In most situations, a daily dose will not exceed 50 mg/kg/day when administered as the monotherapy. In combination, lower dosages of each agent are used and correspondingly, valsartan is given in the range of 1 to 30 mg/kg/day and N-(3-carboxy-1-oxopropyl)-(4S)-p-phenylphenylmethyl)-4-amino-2R-methylbutanoic acid ethyl ester in dosages below 50 mg/kg/day. However, in cases wherein the responder rate is increased with combination treatment, the dosages are identical to those used as monotherapy.

When drugs are administered by oral gavage, the dose of valsartan ranges from 1 to 50 mg/kg/day and N-(3-carboxy-1-oxopropyl)-(4S)-p-phenylphenylmethyl)-4-amino-2R-methylbutanoic acid ethyl ester does not exceed 100 mg/kg/day.

Upon completion of the chronic studies, SHR or DOCA-salt rats are anesthetized and the heart rapidly removed. After separation and removal of the atrial appendages, left ventricle and left plus right ventricle (total) are weighed and recorded. Left ventricular and total ventricular mass are then normalized to body weight and reported. All values reported for blood pressure and cardiac mass represent the group mean ± sem.

Vascular function and structure are evaluated after treatment to assess the beneficial effects of the combination. SHR are studied according to the methods described by Intengan HD, Thibault G, Li JS, Schiffrin EL, Circulation 1999, 100 (22): 2267-2275. Similarly, the methodology for assessing vascular function in DOCA-salt rats is described in Intengan HD, Park JB, Schiffrin, EL, Hypertension, 1999,34(4 Part 2): 907-913.

The available results indicate an unexpected therapeutic effect of a combination according to the invention.

In one aspect is the object of this invention to provide a pharmaceutical combination composition, e.g. for the treatment or prevention of hypertension.

A further active ingredient may be a diuretic, especially hydrochlorothiazide.

In this composition, components (i) and (ii) can be obtained and administered together, one after the other or separately in one combined unit dose form or in two separate unit dose forms. The unit dose form may also be a fixed combination.

The composition of the present invention can also be used for the treatment or prevention of a hypertension comprising administering a therapeutically effective amount of combination of components (i) and (ii) and a pharmaceutically acceptable carrier to a mammal in need of such treatment.

A therapeutically effective amount of each of the component of the combination of the present invention may be administered simultaneously or sequentially and in any order.

The corresponding active ingredient or a pharmaceutically acceptable salt thereof may also be used in form of a hydrate or include other solvents used for crystallization.

The pharmaceutical compositions according to the invention can be prepared in a manner known per se and are those suitable for enteral, such as oral or rectal, and parenteral administration to mammals (warm-blooded animals), including man, comprising a therapeutically effective amount of the pharmacologically active compound, alone or in combination with one or more pharmaceutically acceptable carriers, especially suitable for enteral or parenteral application. Typical oral formulations include tablets, capsules, syrups, elixirs and suspensions. Typical injectable formulations include solutions and suspensions.

The typical pharmaceutically acceptable carriers for use in the formulations described above are exemplified by: sugars such as lactose, sucrose, mannitol and sorbitol; starches such as cornstarch, tapioca starch and potato starch; cellulose and derivatives such as sodium carboxymethyl cellulose, ethyl cellulose and methyl cellulose; calcium phosphates such as dicalcium phosphate and tricalcium phosphate; sodium sulfate; calcium sulfate; polyvinylpyrrolidone; polyvinyl alcohol; stearic acid; alkaline earth metal stearates such as magnesium stearate and calcium stearate; stearic acid; vegetable oils such as peanut oil, cottonseed oil, sesame oil, olive oil and corn oil; non-ionic, cationic and anionic surfactants; ethylene glycol polymers; betacyclodextrin; fatty alcohols; and hydrolyzed cereal solids, as well as other non-toxic compatible fillers, binders, disintegrants, buffers, preservatives, antioxidants, lubricants, flavoring agents, and the like commonly used in pharmaceutical formulations.

The invention also relates to combining separate pharmaceutical compositions in kit form. That is a kit combining two separate units: a valsartan pharmaceutical composition and a NEP inhibitor pharmaceutical composition. The kit form is particularly advantageous when the separate components must be administered in different dosage forms (e.g. parenteral valsartan formulation and oral NEP formulation) or are administered at different dosage intervals.

These pharmaceutical preparations are for enteral, such as oral, and also rectal or parenteral, administration to homeotherms, with the preparations comprising the pharmacological active compound either alone or together with customary pharmaceutical auxiliary substances. For example, the pharmaceutical preparations consist of from 0.1 % to 90 %, preferably of from 1 % to 80 %, of the active compounds. Pharmaceutical preparations for enteral or parenteral administration are, for example, in unit dose forms, such as coated tablets, tablets, capsules or suppositories and also ampoules. These are prepared in a manner which is known per se, for example using conventional mixing, granulation, coating, solubulizing or lyophilizing processes. Thus, pharmaceutical preparations for oral use can be obtained by combining the active compounds with solid excipients, if desired granulating a mixture which has been obtained, and, if required or necessary, processing the mixture or granulate into tablets or coated tablet cores after having added suitable auxiliary substances.

The dosage of the active compound can depend on a variety of factors, such as mode of administration, homeothermic species, age and/or individual condition.

Preferred dosages for the active ingredients of the pharmaceutical combination according to the present invention are therapeutically effective dosages, especially those which are commercially available.

Normally, in the case of oral administration, an approximate daily dose of from 1 mg to 360 mg is to be estimated e.g. for a patient of approximately 75 kg in weight.

Valsartan is supplied in the form of suitable dosage unit form, for example, a capsule or tablet, and comprising a therapeutically effective amount, e.g. from 20 to 320 mg, of valsartan which may be applied to patients. The application of the active ingredient may occur up to three times a day, starting e.g. with a daily dose of 20 mg or 40 mg of valsartan, increasing via 80 mg daily and further to 160 mg daily up to 320 mg daily. Preferably, valsartan is applied once a day or twice a day in heart failure patients with a dose of 80 mg or 160 mg, respectively, each. Corresponding doses may be taken, for example, in the morning, at mid-day or in the evening. Preferred is q.d. or b.i.d. administration in heart failure.

In case of NEP inhibitors, preferred dosage unit forms are, for example, tablets or capsules comprising e.g. from 20 mg to 800 mg, preferably from 50 mg to 700 mg, even more preferably from 100 mg to 600 mg and even more preferably from 100 mg to 300 mg, administered once a day.

In case of diuretics, preferred dosage unit forms are, for example, tablets or capsules comprising e.g. from 5 mg to 50 mg, preferably from 6.25 mg to 25 mg. A daily dose of 6.25 mg, 12.5 mg or 25 mg of hydrochlorothiazide is preferably administered once a day.

The above doses encompass a therapeutically effective amount of the active ingredients of the present invention.

The following examples illustrate the above-described invention.

### Formulation Example 1:

### Film-Coated Tablets:

| Components | Composition Per Unit (mg) | Standards |
|---|---|---|
| Granulation | | |
| Valsartan [= active ingredient] | 80.00 | |
| Microcrystalline cellulose/ Avicel PH 102 | 54.00 | NF, Ph. Eur |
| Crospovidone | 20.00 | NF, Ph. Eur |
| Colloidal anhydrous silica / colloidal silicon dioxide / Aerosil 200 | 0.75 | Ph. Eur/ NF |
| Magnesium stearate | 2.5 | NF, Ph. Eur |

| Blending | | |
|---|---|---|
| Colloidal anhydrous silica / colloidal silicon dioxide / Aerosil 200 | 0.75 | Ph- Eur/ NF |
| Magnesium stearate | 2.00 | NF, Ph. Eur |

| Coating | | |
|---|---|---|
| Purified water *) | | |
| DIOLACK pale red 00F34899 | 7.00 | |
| Total tablet mass | 167.00 | |

| | | |
|---|---|---|
| *)Removed during processing. | | |

The film-coated tablet is manufactured e.g. as follows:

A mixture of valsartan, microcrystalline cellulose, crospovidone, part of the colloidal anhydrous silica/colloidal silicon dioxide/Aerosile 200, silicon dioxide and magnesium stearate is premixed in a diffusion mixer and then sieve through a screening mill. The resulting mixture is again pre-mixed in a diffusion mixer, compacted in a roller compactor and then sieve through a screening mill. To the resulting mixture, the rest of the colloidal anhydrous silica/colloidal silicon dioxide/Aerosile 200 are added and the final blend is made in a diffusion mixer. The whole mixture is compressed in a rotary tabletting machine and the tablets are coated with a film by using Diolack pale red in a perforated pan.

### Formulation Example 2:

### Film-coated tablets:

| Components | Composition Per Unit (mg) | Standards |
|---|---|---|
| Granulation | | |
| Valsartan [= active ingredient] | 160.00 | |
| Microcrystalline cellulose/ Avicel PH 102 | 108.00 | NF, Ph. Eur |
| Crospovidone | 40.00 | NF, Ph. Eur |
| Colloidal anhydrous silica / colloidal silicon dioxide / Aerosil 200 | 1.50 | Ph. Eur/ NF |
| Magnesium stearate | 5.00 | NF, Ph. Eur |

| Blending | | |
|---|---|---|
| Colloidal anhydrous silica / colloidal silicon dioxide / Aerosil 200 | 1.50 | Ph. Eur/ NF |
| Magnesium stearate | 4.00 | NF, Ph. Eur |

| Coating | | |
|---|---|---|
| Opadry Light Brown 00F33172 | 10.00 | |
| Total tablet mass | 330.00 | |

The film-coated tablet is manufactured e.g. as described in Formulation Example 1.

### Formulation Example 3:

### Film-Coated Tablets:

| Components | Composition Per Unit(mg) | Standars |
|---|---|---|
| Core International phase | | |
| Valsartan [= active ingredient] | 40.00 | |
| Silica, colloidal anhydrous (Colloidal silicon dioxide) [= Glidant] | 1.00 | Ph. Eur, USP/NF |
| Magnesium stearate [= Lubricant] | 2.00 | USP/NF |
| Crospovidone [Disintegrant] | 20.00 | Ph. Eur |
| Microcrystalline cellulose [= Binding agent] | 124.00 | USP/NF |

| External phase | | |
|---|---|---|
| Silica, colloidal anhydrous, (Colloidal silicon dioxide) [= Glidant] | 1.00 | Ph. Eur, USP/NF |
| Magnesium stearate [Lubricant] | 2.00 | USP/NF |

| Film coating | | |
|---|---|---|
| Opadry^{®} brown OOF 16711*) | 9.40 | |
| Purified Water **) | | |
| Total tablet mass | 199.44 | |

| | | |
|---|---|---|
| *) The composition of the Opadry^{®} brown OOF16711 coloring agent is tabulated below. **) Removed during processing | | |

Opadry^{®} Composition:

| Ingredient | Approximate % Composition |
|---|---|
| Iron oxide, black (C.I. No. 77499, E 172) | 0.50 |
| Iron oxide, brown (C.I. No. 77499, E 172 | 0.50 |
| Iron oxide, red (C.I. No. 77491, E 172) | 0.50 |
| Iron oxide, yellow (C.I. No. 77492, E 172) | 0.50 |
| Macrogolum (Ph. Eur) | 4.00 |
| Titanium dioxide (C.I. No. 77891, E 171) | 14.00 |
| Hypromellose (Ph. Eur) | 80.00 |

The film-coated tablet is manufactured e.g. as described in Formulation Example 1.

### Formulation Example 4:

### Capsules:

| Components | Composition Per Unit (mg) |
|---|---|
| Valsartan [= active ingredient] | 80.00 |
| Microcrystalline cellulose | 25.10 |
| Crospovidone | 13.00 |
| Povidone | 12.50 |
| Magnesium stearate | 1.30 |
| Sodium lauryl sulphate | 0.60 |

| Shell | |
|---|---|
| Iron oxide, red (C.I. No. 77491, EC No. E 172) | 0.123 |
| Iron oxide, yellow (C.I. No. 77492, EC No. E 172) | 0.123 |
| Iron oxide, black (C.I. No. 77499, EC No. E 172) | 0.245 |
| Titanium dioxide | 1.540 |
| Gelatin | 74.969 |
| Total tablet mass | 209.50 |

### The tablet is manufactured e.g. as follows:

### Granulation/Drying

Valsartan and microcrystallin cellulose are spray-granulated in a fluidized bed granulator with a granulating solution consisting of povidone and sodium lauryl sulphate dissolved in purified water. The granulate obtained is dried in a fluidized bed dryer.

### Milling/Blending

The dried granulate is milled together with crospovidone and magnesium stearate. The mass is then blended in a conical srew type mixer for approximately 10 minutes.

### Encapsulation

The empty hard gelatin capsules are filled with the blended bulk granules under controlled temperature and humidity conditions. The filed capsules are dedusted, visually inspected, weightchecked and quarantined until by Quality assurance department.

### Formulation Example 5:

### Capsules:

| Components | Composition Per Unit (mg) |
|---|---|
| Valsartan [= active ingredient] | 160.00 |
| Microcrystalline cellulose | 50.20 |
| Crospovidone | 26.00 |
| Povidone | 25.00 |
| Magnesium stearate | 2.60 |
| Sodium lauryl sulphate | 1.20 |

| Shell | |
|---|---|
| Iron oxide, red (C.l. No. 77491, EC No. E 172) | 0.123 |
| Iron oxide, yellow (C.l. No. 77492, EC No. E 172) | 0.123 |
| Iron oxide, black (C.l. No. 77499, EC No. E 172) | 0.245 |
| Titanium dioxide | 1.540 |
| Gelatin | 74.969 |
| Total tablet mass | 342.00 |

The formulation is manufactured e.g. as described in Formulation Example 4.

### Formulation Example 6:

### Hard Gelatine Capsule:

| Components | Composition Per Unit (mg) |
|---|---|
| Valsartan [= active ingredient] | 80.00 |
| Sodium laurylsulphate | 0.60 |
| Magnesium stearate | 1.30 |
| Povidone | 12.50 |
| Crospovidone | 13.00 |
| Microcrystalline cellulose | 21.10 |
| Total tablet mass | 130.00 |

### Formulation Example 7:

A hard gelatin capsule, comprising as active ingredient e.g. (S)-N-(1-carboxy-2-methylprop-1-yl)-N-pentanoyl-N-[2'(1 H-tetrazol-5-yl)biphenyl-4-yl-methyl]amine, can be formulated, for example, as follows:

### Composition:

| | |
|---|---|
| (1) valsartan | 80.0 mg |
| (2) microcrystalline cellulose | 110.0 mg |
| (3) polyvidone K30 | 45.2 mg |
| (4) sodium lauryl sulfate | 1.2 mg |
| (5) crospovidone | 26.0 mg |
| (6) magnesium stearate | 2.6 mg |

Components (1) and (2) are granulated with a solution of components (3) and (4) in water. The components (5) and (6) are added to the dry granulate and the mixture is filled into size 1 hard gelatin capsules.

## Claims

1. A pharmaceutical composition comprising
(i) the AT 1-antagonist valsartan or a pharmaceutically acceptable salt the eof and
(ii) N-(3-carboxy-1-oxopropyl)-(4S)-p-phenylphenylmethyl)-4-amino-2R-methylbutanoic acid ethyl ester or N-(3-carboxy-1-oxopropyl)-(4S)-p-phenylphenylmethyl)-4-amino-2R-methylbutanoic acid or a pharmaceutically acceptable salt thereof
and a pharmaceutically acceptable carrier.

2. The pharmaceutical composition of claim 1 wherein N-(3-carboxy-1-oxopropyl)-(4S)-p-phenylphenylmethyl)-4-amino-2R-methylbutanoic acid ethyl ester is a trieathanolamine or tris(hydroxymethyl)aminomethane salt thereof.

3. The pharmaceutical composition of claim 1 further comprising a diuretic.

4. A kit comprising in separate containers in a single package pharmaceutical compositions comprising in one container a pharmaceutical composition comprising N-(3-carboxy-1-oxopropyl)-(4S)-p-phenylphenylmethyl)-4-amino-2R-methylbutanoic acid ethyl ester or N-(3-carboxy-1-oxopropyl)-(4S)-p-phenylphenylmethyl)-4-amino-2R-methylbutanoic acid and in a second container a pharmaceutical composition comprising valsartan.

## Patentansprüche

1. Pharmazeutische Zusammensetzung, umfassend
(i) den AT 1-Antagonist Valsartan oder ein pharmazeutisch akzeptables Salz hiervon und
(ii) N-(3-Carboxy-1-oxopropyl)-(4S)-p-phenylphenylmethyl)-4-amino-2R-methylbutansäureethylester oder N-(3-Carboxy-1-oxopropyl)-(4S)-p-phenylphenylmethyl)-4-amino-2R-methylbutansäure oder ein pharmazeutisch akzeptables Salz hiervon
und einen pharmazeutisch akzeptablen Träger.

2. Pharmazeutische Zusammensetzung nach Anspruch 1, worin der N-(3-Carboxy-1-oxopropyl)-(4S)-p-phenylphenylmethyl)-4-amino-2R-methylbutansäureethylester ein Triethanolaminsalz oder Tris(hydroxymethyl)aminomethansalz hiervon ist.

3. Pharmazeutische Zusammensetzung nach Anspruch 1, weiter umfassend ein Diuretikum.

4. Satz aus zwei getrennten Behältnissen in einer einzelnen Packung für pharmazeutische Zusammensetzungen, der in einem ersten Behältnis eine pharmazeutische Zusammensetzung mit N-(3-Carboxy-1-oxopropyl)-(4S)-p-phenylphenylmethyl)-4-amino-2R-methylbutansäureethylester oder N-(3-Carboxy-1-oxopropyl)-(4S)-p-phenylphenylmethyl)-4-amino-2R-methylbutansäure als Wirkstoff und in einem zweiten Behältnis eine pharmazeutische Zusammensetzung mit Valsartan als Wirkstoff enthält.

## Revendications

1. Composition pharmaceutique comprenant:
(i) l'antagoniste d'AT 1 valsartan ou l'un de ses sels pharmaceutiquement acceptables et
(ii) l'ester éthylique d'acide N-(3-carboxy-1-oxopropyl)-(4S)-p-phénylphénylméthyl)-4-amino-2R-méthylbutanoïque ou l'acide N-(3-carboxy-1-oxopropyl)-(4S)-p-phénylphénylméthyl)-4-amino-2R-méthylbutanoïque ou l'un de leurs sels pharmaceutiquement acceptables, et un véhicule pharmaceutiquement acceptable.

2. Composition pharmaceutique selon la revendication 1, dans laquelle l'ester éthylique d'acide N-(3-carboxy-1-oxopropyl)-(4S)-p-phénylphénylméthyl)-4-amino-2R-méthylbutanoïque est un sel de triéthanolamine ou de tris(hydroxyméthyl)aminométhane de celui-ci.

3. Composition pharmaceutique selon la revendication 1, comprenant en outre un diurétique.

4. Trousse comprenant, dans des récipients séparés, dans un seul conditionnement, des compositions pharmaceutiques comprenant, dans un récipient, une composition pharmaceutique comprenant l'ester éthylique d'acide N-(3-carboxy-1-oxopropyl)-(4S)-p-phénylphénylméthyl)-4-amino-2R-méthylbutanoïque ou l'acide N-(3-carboxy-1-oxopropyl)-(4S)-p-phénylphénylméthyl)-4-amino-2R-méthylbutanoïque et, dans un deuxième récipient, une composition pharmaceutique comprenant du valsartan.
